(19) Europäisches Patentamt
European Patent Office
Office européen des brevets

(11) Veröffentlichungsnummer : **0 021 215**
**B1**

(12) # EUROPÄISCHE PATENTSCHRIFT

(45) Veröffentlichungstag der Patentschrift : **13.07.83**

(51) Int. Cl.³ : **C 07 H 15/22**, A 61 K 31/70

(21) Anmeldenummer : **80103193.1**

(22) Anmeldetag : **09.06.80**

(54) **Pseudotrisaccharide, ihre Herstellung und Verwendung als Arzneimittel.**

(30) Priorität : **19.06.79 DE 2924659**

(43) Veröffentlichungstag der Anmeldung : **07.01.81 Patentblatt 81/01**

(45) Bekanntmachung des Hinweises auf die Patenterteilung : **13.07.83 Patentblatt 83/28**

(84) Benannte Vertragsstaaten : **AT BE CH DE FR GB IT LI NL SE**

(56) Entgegenhaltungen :
**EP A 0 007 996**
**DE A 2 708 008**
**FR A 2 292 482**
**FR A 2 305 190**
**FR A 2 348 223**
**FR A 2 383 963**

**CHEMICAL ABSTRACTS, Band 87, Nr. 7, 15. August 1977, Seite 503, Nr. 53534e**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Stadler, Peter, Dr.**
**Am Ideck 8**
**D-5657 Haan (DE)**
Erfinder : **Metzger, Karl Georg, Dr.**
**Pahlkestrasse 15**
**D-5600 Wuppertal 1 (DE)**
Erfinder : **Voss, Eckart, Dr.**
**Johann-Janssen-Strasse 38**
**D-5090 Leverkusen 1 (DE)**
Erfinder : **Petersen, Uwe, Dr.**
**Auf dem Forst 4**
**D-5090 Leverkusen 1 (DE)**
Erfinder : **Zeiler, Hans-Joachim, Dr.**
**Windratherstrasse 188**
**D-5620 Velbert 15 (DE)**

**EP 0 021 215 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Pseudotrisaccharide, ihre Herstellung und Verwendung als Arzneimittel

Die Erfindung betrifft neue Pseudotrisaccharide, Verfahren zu ihrer Herstellung sowie ihre Verwendung als Arzneimittel.

Im besonderen bezieht sich die Erfindung auf neue, antibakteriell wirksame Aminoglykosidantibiotika vom Typ der 4,6-Di-O-(aminoglykosyl)-1,3-diaminocyclitole.

Aminoglykosidantibiotika sind wichtige Substanzen zur wirkungsvollen Bekämpfung bakterieller Infektionen. Das Auftreten resistenter Keime mindert jedoch in vielen Fällen ihre breite Anwendbarkeit ; des weiteren können Nebenwirkungen, wie etwa Oto- und Nephrotoxizität auftreten. Durch Derivatisierung gelingt es in einigen Fällen, diese Nachteile zu beseitigen.

Die erfindungsgemäßen Pseudotrisaccharide lassen sich durch Formel (I) wiedergeben,

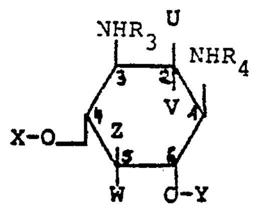

(I)

in der

X einen Rest

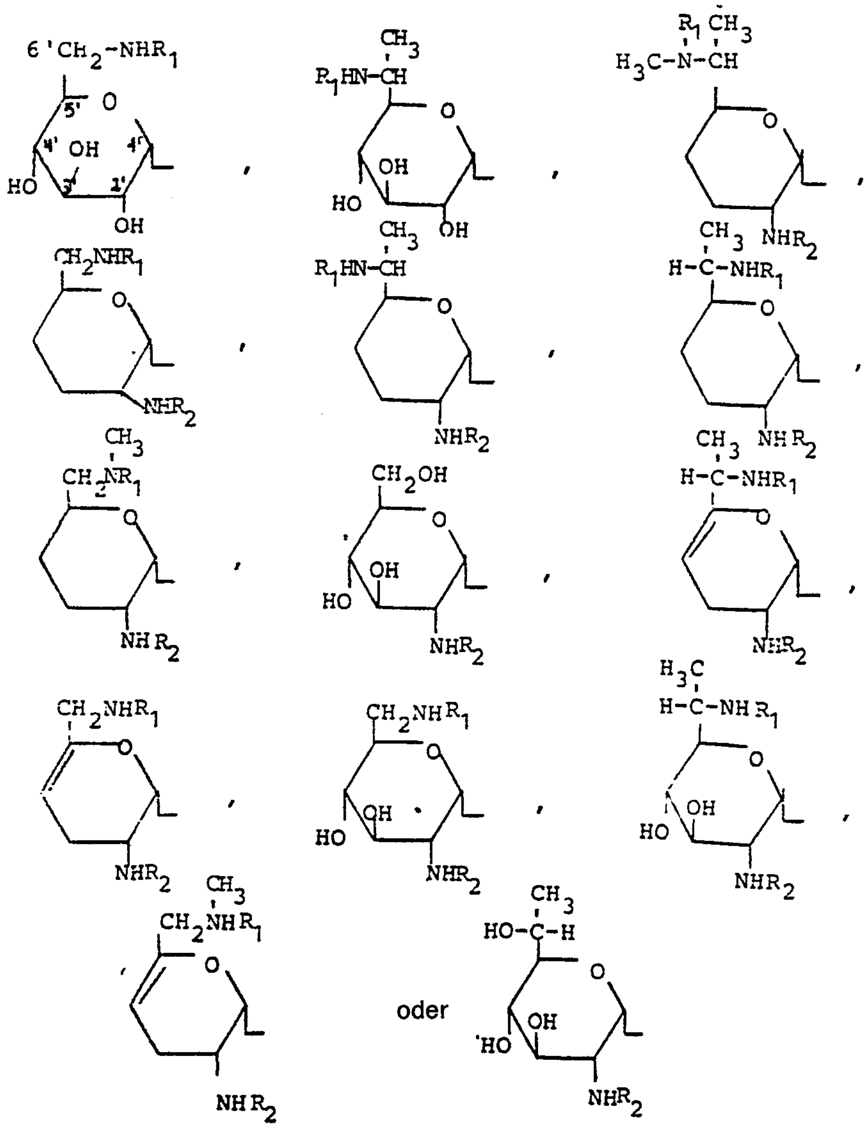

oder

Y einen Rest

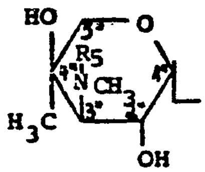
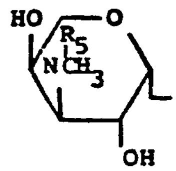
oder
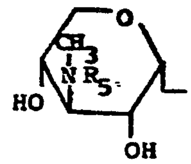

U und V beide Wasserstoff oder einer Wasserstoff und einer Hydroxy,

W und Z beide Wasserstoff oder einer Wasserstoff und einer Hydroxy oder Z $NHR_6$ und W Wasserstoff und

worin $R_1$, $R_2$, $R_3$, $R_4$, $R_5$ und $R_6$ unabhängig voneinander Wasserstoff oder einen Rest der Formel

$$CH_2-(CH_2)_{n_1}-(\underset{BR_7}{CH})_{n_2}-A_{n_3}-(\underset{BR_7}{CH})_{n_4}-CH_2R_8$$

bedeuten, wobei

A für

$$\left[-\underset{(CH_2BR_7)_{n_5}}{CH}\!-\!(2-n_5)\right]$$

$R_7$ für Wasserstoff, Alkyl oder Acyl, oder 2 Reste $R_7$ gemeinsam für Alkyliden

$R_8$ für Wasserstoff oder $BR_7$,

und worin

$R_1$, $R_2$, $R_3$, $R_4$, $R_5$ und $R_6$ unabhängig voneinander Wasserstoff oder einen durch reduktive Alkylierung in Gegenwart einer Amino-desoxy-aldose eingeführten Aminopolyhydroxyalkylrest bedeuten, der nicht mehr als 2 Aminogruppen enthält und welcher gegebenenfalls am N-Atom acyliert oder sulphenylier ist sowie gegebenenfalls an einer oder mehreren OH-Gruppen Alkyl-, Acyl- oder Alkylidenreste trägt, mit der Bedingung, daß wenigstens einer der Reste $R_1$ bis $R_5$ nicht Wasserstoff ist.

Von besonderem Interesse sind die erfindungsgemäßen Aminoglykosidantibiotika gemäß Formel (I), die von den Antibiotika Gentamicin A, Gentamicin B, Gentamicin $B_1$, Gentamicin $C_1$, Gentamicin $C_{1a}$, Gentamicin $C_2$, Gentamicin $C_{2a}$, Gentamicin $C_{2b}$, Gentamicin $X_2$, Sisomicin, JI-20A, JI-20B, Verdamicin G52, G418, 66-40D, Mutamicin 1, Mutamicin 2, Mutamicin 4, Mutamicin 5 und Mutamicin 6 abgeleitet sind.

Von diesen sind die durch Formel (II) wiedergegebenen Sisomicinderivate

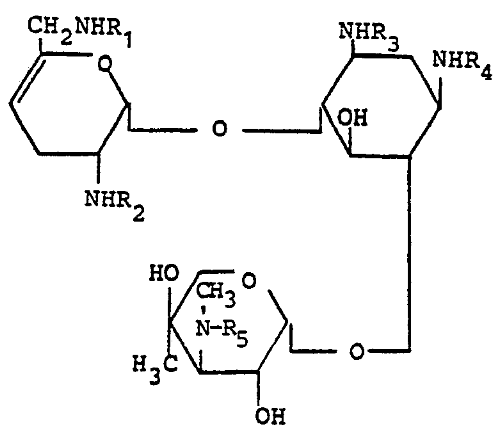

(II)

bei denen $R_1$, $R_2$, $R_3$, $R_4$ und $R_5$ die oben angegebene Bedeutung besitzen, besonders wertvoll.

Alkyl $R_7$ ist insbesondere $C_1$-$C_4$-Alkyl, Acyl $R_7$ ist insbesondere $C_1$-$C_4$-Alkylcarbonyl, Formyl oder Benzoyl.

Alkyliden, gebildet von 2 Resten $R_7$ ist insbesondere $C_1$-$C_6$-Alkyliden.

Bevorzugte Verbindungen innerhalb der Formel (I) sind solche, in denen $R_4$ und einer der Reste $R_1$ und $R_2$ nicht Wasserstoff sind und die Reste $R_3$, $R_5$, $R_6$ und der andere der Reste $R_1$ und $R_2$ Wasserstoff bedeuten. Ganz besonders bevorzugte Verbindungen sind solche, in denen $R_1$, $R_2$, $R_3$, $R_5$ und $R_6$ Wasserstoff bedeuten und $R_4$ nicht Wasserstoff ist.

Innerhalb dieser Verbindungen sind solche Verbindungen von besonderem Interesse, in denen $n_3$ und $n_4$ O bedeuten und die Summe aus $n_1$ und $n_2$ 1, 2 oder 3 beträgt.

Die erfindungsgemäßen Verbindungen sowie pharmazeutisch verwendbare Salze zeigen starke antibakterielle Eigenschaften gegen eine Vielzahl von Keimen und eine außergewöhnlich gute Verträglichkeit.

Die pharmazeutisch verwendbaren Salze leiten sich insbesondere von anorganischen oder organischen Säuren wie Schwefelsäure, Phosphorsäure, Salpetersäure, Salzsäure, Bromwasserstoffsäure, Essigsäure, Propionsäure, Ascorbinsäure, Zitronensäure usw. ab.

Geeignete Reste $R_1$ bis $R_6$ sind beispielsweise geradkettige Aminopolyhydroxyalkylreste wie 2-Amino-3,4,5,6-tetrahydroxyhexyl, 3-Amino-2,4,5,6-tetrahydroxyhexyl, 4-Amino-2,3,5,6-tetrahydroxyhexyl, 5-Amino-2,3,4,6-tetrahydroxyhexyl, 6-Amino-2,3,4,5-tetrahydroxyhexyl, 2-Amino-3,4,5-trihydroxypentyl, 3-Amino-2,4,5-trihydroxypentyl, 4-Amino-2,3,5-trihydroxypentyl, 5-Amino-2,3,4-trihydroxypentyl, 3-Amino-2, 4-dihydroxybutyl, 4-Amino-2,3-dihydroxybutyl, 2-Amino-3,4,5-trihydroxyhexyl, 3-Amino-2,4,5-trihydroxyhexyl, 4-Amino-2,3,5-trihydroxyhexyl, 5-Amino-2,3,4-trihydroxyhexyl, 3-Amino-4,5-dihydroxypentyl oder 3-Amino-4,5-dihydroxyhexyl, Diaminopolyhydroxyalkylreste wie 2,3-Diamino-4,5,6-trihydroxyhexyl, 3,6-Diamino-2,4,5-trihydroxyhexyl oder 2,3-Diamino-4,5-dihydroxypentyl sowie N-alkylierte oder N-acylierte Aminopolyhydroxyalkylreste wie z. B. 2-Methylamino-3,4,5,6-tetrahydroxyhexyl, 3-Methylamino-2,4,5,6-tetrahydroxyhexyl, 5-Methylamino-2,3,4,6-tetrahydroxyhexyl, 2-Methylamino-3,4,5-trihydroxypentyl, 4-Methylamino-2,3,5-trihydroxypentyl, 5-Methylamino-2,3,4-trihydroxypentyl, 6-Hydroxyethylamino-2,3,4,5-tetrahydroxyhexyl, 3-Dimethylamino-2,5-dihydroxyhexyl, 2-Acetylamino-3,4,5,6-tetrahydroxylhexyl, 2-Ethyloxycarbonylamino-3,4,5,6-tetrahydroxyhexyl, 3-Acetylamino-2,4,5-trihydroxypentyl oder 5-Ethylamino-1,3,4-trihydroxypentyl und weiterhin O-alkylierte Aminopolyhydroxyreste wie z. B. 5-Amino-2,3,4-trihydroxy-6-methoxyhexyl.

Die vorstehend aufgeführten Reste sind lediglich beispielhaft zu verstehen. Sie enthalten alle mindestens ein — in den meisten Fällen mehrere — chirale C-Atome und liegen als optisch reine Diastereomere oder Diasteromerengemische vor. Es kann von Vorteil sein, die erfindungsgemäßen Verbindungen als optisch reine Produkte zu verwenden.

Als Beispiele für die erfindungsgemäßen Wirkstoffe seien im einzelnen genannt :

1-N-[(S,S,S,R)-2-Amino-3,4,5,6-tetrahydroxyhexyl]-sisomicin, 1-N-[(R,S,S,R)-, 1-N[(S,R,S,R)-,

1-N-[R,S,R,S]-, 1-N-[(R,R,S,R)- und 1-N[(S,R,R,R)-2-Amino-3,4,5,6-1-N-[S,S,S,R)-3-Amino-2,4,5,6-tetrahydroxyhexyl]-sisomicin, 1-N[(S,S,R,S)- und 1-N[(S,S,S,R)-4-Amino-2,3,5,6-tetrahydroxyhexyl]-sisomicin,

1-N-[(S,R,R,R)- und 1-N-[(S,R,R,S)-5-Amino-2,3,4,6-tetrahydroxyhexyl]-sisomicin, 1-N-[1-N-[(S,R,R,R)-,

1-N-[(S,R,R,R)-, 1-N-[(S,R,R,R)-, 1-N-[(S,R,S,R)- und

1-N-[(R,R,R,R)-6-Amino-2,3,4,5-tetrahydroxyhexyl)-sisomicin, 1-N-[(S,S,R)-, 1-N-[R,R,S)-, 1-N-[R,S,R)-,

1-N-[(S,R,S)-, 1-N-[(S,R,R)- und 1-N-[(R,R,R)-2-Amino-3,4,5-trihydroxypentyl]-sisomicin, 1-N-[(S,R,S)-,

1-N-[(S,S,R)-, 1-N-[(S,R,R)- und 1-N-[(R,S,S)-3-Amino-2,4,5-trihydroxypentyl]-sisomicin, 1-N-[(S,R,S)-4-Amino-2,3,5-trihydroxypentyl]-sisomicin, 1-N-[(S,S,S)-1-N-[(S,R,R)-, 1-N-[(S,S,R)- und 1-N-[S,S,R)- und 1-N-[(S,R,S)-5-Amino-2,3,4-trihydroxypentyl]-sisomicin,

1-N-[(S,S,R,R)-, 1-N-[(S,R,R,R)-2-Amino-3,4,5-trihydroxyhexyl]-sisomicin, 1-N-[(S,S,R,R)-4-Amino-2,3, 5-trihydroxyhexyl]-sisomicin, 1-N-[(S,R,S,R)-3-Amino-2,4,5-trihydroxyhexyl]-sisomicin, 1-N-[(S,S,R,S)-5-Amino-2,3,4-trihydroxyhexyl]-sisomicin und 1-N-[(S,S,S)-6-Amino-2,3,4-trihydroxyhexyl]-sisomicin, 1-N-[(S,S)-3-Amino-4,5-dihydroxypentyl]-sisomicin, 1-N-[(S,R,S,R)-2,3-diamino-4,5,6-trihydroxyhexyl]-sisomicin, 1-N-[(S,R,R,R)-2,6-diamino-3,4,5-trihydroxyhexyl]-sisomicin,

1-N-[(S,S,S,R)-3,6-Diamino-2,4,5-trihydroxyhexyl]-sisomicin, 1-N-[(S,R,S,S)-2,4-Diamino-3,5,6-trihydroxyhexyl]-sisomicin, 1-N-[(R,R,R)-2,3-Diamino-4,5-dihydroxypentyl]-sisomicin, 1-N-[S,R,S,R)-2-Methylamino-3,4,5,6-tetrahydroxyhexyl]-sisomicin, 1-N-[(S,R,S,R)-3-Methylamino-2,3,4,6-tetrahydroxyhexyl]-sisomicin,

1-N-[(S,R,R,S)-5-Methylamino-2,3,4,6-tetrahydroxyhexyl]-sisomicin, 1-N-[(S,R,S,R)-6-Hydroxyethylamino-2,3,4,5-tetrahydroxyhexyl]-sisomicin, 1-N-[(S,S,R)-2-Methylamino-3,4,5-trihydroxypentyl]-sisomicin,

1-N-[(S,S,S)-3-Methylamino-2,4,5-trihydroxypentyl]-sisomicin, 1-N-[(S,R,S)-4-Methylamino-2,3,5-trihydroxypentyl]-sisomicin, 1-N-[(S,R,R)-5-Methylamino-2,3,4-trihydroxypentyl]-sisomicin, 1-N-[(S,S,R)-3-Dimethylamino-2,5-dihydroxypentyl]-sisomicin, 1-N-[(S,R,S,R)-2-Ethoxycarbonylamino-3,4,5,6-tetrahydroxyhexyl]-sisomicin, 1-N-[(S,R,S,R)-2-Acetylamino-3,4,5,6-tetrahydroxyhexyl]-sisomicin, 1-N-[(S,R,S, R)-6-Acetylamino-2,3,4,5-tetrahydroxyhexyl]-sisomicin,

1-N-[(S,S,R)-3-Acetylamino-2,4,5-trihydroxypentyl]-sisomicin, 1-N-(S,R,S)-4-Acetylamino-2,3,5-trihydroxypentyl]-sisomicin und 1-N-[(S,S,R)-5-Acetylamino-2,3,4-trihydroxypentyl]-sisomicin, 1-N-[(S,R,R, R)-5-Amino-2,3,4-trihydroxy-6-methoxyhexyl]-sisomicin,

1-N-[(S,S,R)-3-amino-2,4,5-trihydroxypentyl]-6′-N-hydroxyethyl-sisomicin, 1-N-[(S,R,S)-4-Amino-2,3, 5-trihydroxypentyl]-6′N-aminoethyl-sisomicin und 1,6′-Di-N-[(S,R,R)-5-Amino-2,3,4-trihydroxypentyl]-sisomicin.

Die erfindungsgemäßen Verbindungen werden erhalten, indem man selektiv acylierte oder sulfenylierte Verbindungen oder Verbindungen die gleichzeitig Acyl- und Sulfenylgruppen tragen der Formel I, worin

$R_1$, $R_2$, $R_3$, $R_4$, $R_5$ und $R_6$ Wasserstoff, —SR′ oder —CO—A′ bezeichnen mit der Maßgabe, daß wenigstens einer der Reste $R_1$ bis $R_6$ Wasserstoff bedeutet und wenigstens einer der Reste $R_1$ bis

$R_6$ —SR′ oder —CO—A′ bedeutet,
wobei R′ gegebenenfalls substituiertes Phenyl, Di- oder Triphenylmethyl und A′

C $Hal_3$, $-(CH_2)_{n_6}-D$ oder 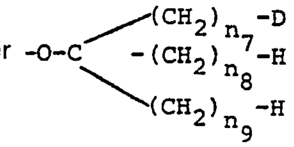

bedeuten, worin Hal für Fluor, Chlor oder Brom,
D für Wasserstoff oder gegebenenfalls substituiertes Phenyl und
$n_6$, $n_7$, $n_8$ und $n_9$ unabhängig voneinander für eine Zahl von 0 bis 5 stehen,
mit einer Aminodesoxyaldose, welche gegebenenfalls am N-Atom acyliert oder sulphenyliert ist, sowie gegebenenfalls an einer oder mehreren OH-Gruppen (außer an der 1-OH-Gruppe) Alkyl-, Acyl- oder Alkyldidenreste trägt, in Gegenwart eines Wasserstoffdonor-Reduktionsmittels umsetzt und anschließend die Schutzgruppen —SR′ oder —COA′ und gegebenenfalls Acylgruppen abspaltet.

Gegebenenfalls substituiertes Phenyl R′ ist insbesondere Phenyl oder durch ein bis drei Substituenten aus der Reihe Nitro, $C_1$-$C_4$-Alkyl, $C_1$-$C_4$-Alkoxy, $C_1$-$C_4$-Alkoxycarbonyl oder Phenyl oder 1 bis 5 Halogenatome substituiertes Phenyl.

Gegebenenfalls substituiertes Phenyl D ist insbesondere Phenyl oder durch ein oder zwei Substituenten aus der Reihe Nitro, $C_1$-$C_4$-Alkyl, $C_1$-$C_4$-Alkoxy, Phenyl oder Halogen substituiertes Phenyl.

Die Verbindung der Formel I, in denen $R_1$ bis $R_6$ Wasserstoff, SR′ oder CO—A′ bedeuten, werden erhalten, indem man beispielsweise Sisomicin mit o-Nitrophenylsulfenylsäure-p-nitrophenylester in einem inerten Lösungsmittel gegebenenfalls unter Zusatz von Wasser bei Temperaturen zwischen − 30 und + 50 °C in Gegenwart einer Base umsetzt. Dabei setzt man auf ein Mol Sisomicin je nach dem, wieviel Aminogruppen geschützt werden sollen, ein bis vier Mol des Sulfensäureesters ein. Weitere Reagenzien, mit denen Schutzgruppen eingeführt werden können, sind Tritylsulfenylchlorid, o-Nitrophenylsulfenylchlorid, 2,4-Dinitrophenylsulfenylchlorid, 2,4,5-Trichlorphenylsulfenylchlorid, Pentachlorphenylsulfenylchlorid, 2,4-Dinitrophenylsulfensäure-p-nitrophenylester, 2,4,5-Trichlorphenylsulfensäure-p-Nitrophenylester, Pentachlorphenylsulfonsäure-p-nitrophenylester, Acetanhydrid, Trichloracetanhydrid, Acetylchlorid, Di-t-butylpyrocarbonat und Diethylpyrocarbonat.

Die Abspaltung der Sulfenylschutzgruppen kann mit schwachen Säuren erfolgen oder bevorzugt mit Nukleophilen, vorzugsweise mit H-S-Gruppen-haltigen Nukleophilen wie $H_2S$, Thiophenol oder 2-Mercaptobenzthiazol in einem geeigneten organischen Lösungsmittel wie Dichlormethan, Methanol oder auch Gemischen solcher Lösungsmittel. Die Abspaltung der übrigen Schutzgruppen kann mit wäßrigem Alkali- oder Erdalkalihydroxid oder mit Säuren wie Trifluoressigsäure, Perchlorsäure oder Bortrifluoridätherat erfolgen.

Die reduktive Alkylierung mit einem Aldehyd der Formel (III) in Gegenwart eines Wasserstoffdonor-Reduktionsmittels wird je nach Reaktivität des Aldehyds bei Raumtemperatur oder bei erhöhter Temperatur bis etwa 80 °C durchgeführt. Sie kann in Gegenwart von Luft durchgeführt werden, obwohl es günstiger sein kann, die Reaktion unter Inertgas (Argon, Stickstoff) durchzuführen. Durch dünnschichtchromatographische Bestimmungen kann festgestellt werden, wann die Reaktion beendet ist.

Wasserstoff-Donor-Reduktionsmittel, die bei diesem Verfahren Verwendung finden, umfassen Dialkylaminborane, z. B. Dimethylaminboran, Diethylaminboran und Morpholinboran, Tetraalkylammoniumcyanoborhydride, (z. B. Tetrabutylammoniumcyanoborhydrid), Alkalimetallborhydride, z. B. Natriumborhydrid und vorzugsweise Alkalimetallcyanoborhydride, Lithiumcyanoborhydrid und Natriumcyanoborhydrid.

Das Verfahren wird üblicherweise in einem inerten Lösungsmittel durchgeführt. Das Lösungsmittel kann ein organisches oder anorganisches sein, in dem das selektiv geschützte 4,6-Di-O-(aminoglycosyl)-1,3-diaminocyclitol und die anderen Reagentien löslich sind und das unter den Reaktionsbedingungen nach Möglichkeit Nebenreaktionen herabsetzt oder verhindert. Obwohl wasserfreie aprotische Lösungsmittel mit Vorteil eingesetzt werden können, z. B. Tetrahydrofuran wenn das Reduktionsmittel Morpholinboran ist, wird gewöhnlich doch ein protisches Lösungsmittel verwendet. Als solches eignet sich z. B. ein niederes Alkanol oder vorzugsweise Wasser oder ein wäßriges niedriges Alkanol, vorzugsweise wäßriges Methanol, Ethanol oder Aceton oder andere Lösungsmittelsysteme, die Wasser enthalten wie wäßriges Dimethylformamid, wäßriges Hexamethylphosphoramid, wäßriges Tetrahydrofuran oder wäßriger Ethylenglycoldimethylether.

Das Verfahren wird gewöhnlich in einem pH-Bereich von 1 bis 11 und vorzugsweise bei pH 3,5 bis 8 durchgeführt.

Die im erfindungsgemäßen Verfahren verwendeten Aminozucker bzw. deren Derivate sind an sich bekannt, bzw. durch bekannte Synthesen zugänglich, wie sie z. B. in « Methods in Carbohydrate Chemistry », Academic Press — New York and London — Band I, 206-257 (1962) und Band VI, 208-282 (1972) beschrieben werden. Sie können entweder in freier Form oder auch als Acetale, z. B. als Dimethylacetale, zur reduktiven Alkylierung eingesetzt werden.

Man arbeitet bei der Verwendung von Acetalen in Gegenwart von Mineralsäuren oder organischen

Säuren wie Essigsäure, wodurch das Acetal gespalten wird und der freigesetzte Aldehyd sofort mit der entsprechenden Aminogruppe der Aminotrisaccharidderivate reagiert.

Ein wichtiger Vorteil der erfindungsgemäßen Verwendung von Kohlenhydraten oder deren Derivaten für die reduktive Einführung von Aminopolyhydroxyalkylresten ist die Tatsache, daß in Form der Aminozucker bzw. deren N-geschützter Derivate eine große Zahl polyfunktioneller und vor allem optisch einheitlicher Aldehydverbindungen für reduktive Alkylierungen zur Verfügung stehen. Dabei ist besonders zu berücksichtigen, daß die reinen Komponenten von Diastereomerengemischen der erfindungsgemäßen Aminoglykosidantibiotika sich in ihren biologischen Eigenschaften meist deutlich voneinander unterscheiden.

Bei der erfindungsgemäß verwendeten N-blockierten Aminozuckern handelt es sich um N-acylierte oder N-sulfenylierte Derivate von z. B. 4-Amino-D-erythrose, 4-Amino-4-desoxy-L-lyxose, 3-Amino-3-desoxy-D-ribose, 2-Amino-2-desoxy-D-ribose, 6-Amino-6-desoxy-D-glucose, 5-Amino-5-desoxy-L-idose, 4-Amino-4-desoxy-D-galactose, 3-Amino-3-desoxy-D-glucose, 2-Amino-2-desoxy-D-mannose, 3-Amino-2, 3-didesoxy-D-ribose, 2,6-Diamino-2,6-didesoxy-D-glucose, 3,6-Diamino-3,6-didesoxy-D-allose, 2-Methylamino-2-desoxy-D-glucose, 3-Methylamino-3-desoxy-D-glucose, 3-Methylamino-3-desoxy-D-ribose oder 5-Methylamino-5-desoxy-D-ribose.

Die N-acylierten oder N-sulfenylierten Verbindungen werden entsprechend den Angaben auf S.11, 1. Absatz hergestellt.

Die vorstehend genannten Verbindungen stellen nur eine Auswahl dar, die das erfindungsgemäße Verfahren erläutern soll.

Verwendet man N-blockierte Aminozucker, welche an einer oder mehreren OH-Gruppen, außer an der 1-OH-Gruppe-Alkyl-, Acyl- oder Alkylidenreste tragen, kommt man nach Abspaltung der Aminoschutzgruppen zu Verbindungen der Formel I mit Alkoxy-, Acyloxy oder O-Alkylidenresten in der neu eingeführten Gruppe R. Beispiele für solche Aminozuckerderivate sind N-blockierte 2-Amino-2-desoxy-6-o-methyl-D-glucose, 5-Amino-5-desoxy-2,3-0-isopropyliden-D-ribose. Als Aminoschutzgruppen werden hierbei die in Formel (II) angegebenen Reste verwendet.

In einer anderen Ausführungsform des erfindungsgemäßen Verfahrens setzt man an den Amino- und gegebenenfalls an den OH-Gruppen selektiv blockierte Aminozuckerderivate, die in der Aldehyd- und nicht in der Halbacetalform vorliegen, als Aldehyd-Komponente zur reduktiven Alkylierung der selektiv geschützten Aminoglycoside ein, spaltet anschließend sämtliche im Molekül vorhandenen O- und N-Schutzgruppen ab und kommt so zu den Verbindungen der Formel (I).

Die vorliegende Erfindung umfaßt auch Verfahren zur Darstellung von N-(Amino-polyhydroxy-alkyl)-aminotrisacchariden der Formel (I), bei welchen man die 4,6-di-O-(Aminoglycosyl)-1,3-diaminocyclitole in ungeschützter Form — d. h. als freie Base oder deren Säureadditionssalze mit den Aminodesoxyaldosen und einem Wasserstoffdonorreduktionsmittel umsetzt.

Dazu werden die entsprechenden Aminotrisaccharide der Formel (I), bei denen in diesem Fall $R_1$ bis $R_6$ für Wasserstoff stehen, oder deren Säureadditionssalze, in welchen ein Teil der im Molekül vorhandenen Aminogruppen durch Mineralsäuren neutralisiert sind, mit 1 bis 2 äquivalentem N-blockiertem Aminozucker in Gegenwart eines Wasserstoffdonorreduktionsmittels wie Natriumcyanoborhydrid oder Dimethylaminboran in einem geeigneten Lösungsmittel umgesetzt. Nach der Reaktion spaltet man die im verwendeten Aminozucker vorhandenen Aminoschutzgruppen in der oben beschriebenen Weise ab und isoliert die N-(Aminopolyhydroxyalkyl)-aminotrisaccharide der Formel (I), wobei in manchen Fällen eine säulenchromatographische Abtrennung der angestrebten Endprodukten von unerwünschten Nebenprodukten notwendig sein kann.

Die erfindungsgemäßen Verbindungen sind antimikrobielle Mittel mit einem breiten Wirkungsspektrum und besonderer Wirksamkeit gegen gram-negative Bakterien, z. B. E. coli, Proteus, Klebsiella und Pseudomonas. Hemmhöfe im Agar-Lochtest wurden z. B. gegen folgende Bakterienstämme bei einer Konzentration von 100 Mikrogramm/1 ml gefunden :

Pseudomonas aerug. 5737
Pseudomonas aerug. F 41
Klebsiella pneum. 2 München
Klebsiella pneum. 1 Düsseldorf
E. coli Münster
E. coli Neumann

Die erfindungsgemäßen Verbindungen werden daher für Arzneimittel verwendet, die bei bakteriellen Infektionen verabreicht werden.

Im allgemeinen hänt die zu verabreichende Dosis der Verbindungen der Erfindung vom Alter und Gewicht des Lebewesens, von der Verabreichungsart, vom Typ und von der Schwere der bakteriellen Infektionen ab. Die Dosierung der erfindungsgemäßen Verbindungen ist gewöhnlich der Dosierung der 1-N- unsubstituierten Verbindungen ähnlich. Der Dosierungsspielraum beträgt von 20 mg/Tag/Mensch bis 2 000/mg/Tag/Mensch, vorzugsweise 100 mg-500 mg/Tag.

Die Verabreichung der Verbindungen der Erfindung kann in Einzelgaben oder auf mehreren Gaben verteilt erfolgen. Sie können auch topisch verabreicht werden in der Form von Salben, Cremen oder

Lotionen. Pharmazeutische Trägerstoffe für diese Formulierungen umfassen Wasser, Öle, Fette, Polyester und Polyole.

Wie im folgenden gezeigt wird ist das Verfahren zur Herstellung von Arzneimitteln, welche die erfindungsgemäßen Produkte enthalten, dadurch gekennzeichnet, daß man die neuen Verbindungen mit pharmazeutisch geeigneten Träger- und/oder Zusatzstoffen vermischt.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5 mal am Tag.

Die antibakterien Mittel der Erfindung können in flüssiger Form als Lösungen oder Suspensionen zur Anwendung an Ohren und Augen oder zur parenteralen Verabreichung in Form intramuskulärer Injektionen vorliegen. Injektionslösungen oder -suspensionen werden gewöhnlich so verabreicht, daß ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierenden Keims und von den individuellen Charakteristika des zu Behandelnden ab.

Formulierung 1

| Tablette | 10 mg Tab. | 25 mg Tab. | 100 mg Tab. |
|---|---|---|---|
| a) Verbindung von Bsp. 8 | 10,50+ mg | 26,25+ mg | 105.00+ mg |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

+ 5 %iger Überschuß

| | 10 mg Tab. | 25 mg Tab. | 100 mg Tab. |
|---|---|---|---|
| b) Verbindung von Bsp. 8 | 10,50+ mg | 26,25+ mg | 105,00+ mg |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

+ 5 %iger Überschuß

Zur Herstellung der Tabletten wird eine Aufschlämmung des betreffenden Wirkstoffs, Lactose und Polyvinylpyrrolidon, hergestellt und diese sprühgetrocknet. Man gibt die Maisstärke und Magnesiumstearat zu und verpreßt zu Tabletten.

Formulierung 2

Salbe

| | |
|---|---|
| Verbindung von Bsp. 8 | 1,0 g |
| Methylparaben U.S.P. | 0,5 g |
| Propylparaben U.S.P. | 0,1 g |
| Petrolatum | auf 1 000 g |

Herstellung

(1) Man schmilzt das Petrolatum,

(2) mischt den Wirkstoff Methylparaben und Propylparaben mit ca. 10 % des geschmolzenen Petrolatum,

(3) gibt das Gemisch in eine Kolloidmühle,

(4) gibt das restliche Petrolatum unter Rühren zu und kühlt, bis es halbfest wird. Das Produkt wird in geeignete Behälter abgefüllt.

Formulierung 3

| Injektionslösung | Per 2,0 ml Viole | per 50 Liter |
|---|---|---|
| Verbindung von Bsp. 8 | 84,0 mg+ | 2 100.0 gm |
| Methylparaben, U.S.P. | 3,6 mg | 90,0 gm |
| Propylparaben, U.S.P. | 0,4 mg | 10,0 gm |
| Natriumbisulfit, U.S.P. | 6,4 mg | 160,0 gm |
| Dinatriumäthylendiamin-tetraacetat-dihydrat | 0,2 mg | 5,0 mg |
| Wasser, U.S.P. q.s. | 2,0 mg | 50,0 Liter |

+ 5 %iger Überschuß

Beispiel 1

1,2',3,6'-Tetra-N-acetyl-sisomicin

1,1 g Sisomicin werden in 120 ml Wasser gelöst. Nach Zusatz von 60 ml Methanol tropft man unter Rühren 2,5 ml Acetanhydrid dazu. Nach 15 Minuten wird im Vakuum zur Trockene eingedampft. Den Rückstand löst man in 10 ml Methanol und tropft diese Lösung in eine Mischung von 30 ml Ether und 30 ml Petrolether wobei das gewünschte Produkt ausfällt. Ausbeute = 1,43 g, MS : m/e = 615.

$^{13}C$-NMR ($CD_3OD$) ;
δ = 50,14 (C-1) ; 49,20 (C-3) ;
46.88 (C-2') ; 42,26 (C-6') ;
173.24, 173.13, 172.63 (>c = 0) ppm.

Beispiel 2

1,3,3",6'-Tetra-N-ethoxycarbonyl-sisomicin

450 mg Sisomicin werden in 10 ml Wasser gelöst. Nach Zugabe von 10 ml Methanol versetzt man unter gutem Rühren mit 870 mg Pyrokohlensäure-di-ethylester. Nach 1,3-Stunden Rühren bei Raumtemperatur versetzt man mit 5 ml Wasser, filtriert und dampft das Filtrat im Vakuum zur Trockene ein. Man löst den Rückstand in Methanol und fällt das gewünschte Produkt durch Zugabe von Ether und Petrolether.

Ausbeute = 600 mg ;
$^{13}C$-NMR ($CD_2OD$) ;
δ = 66.01 (c-3") ; 52.23 (c-1) ;
51.67 (C-3) ; 48.23 (c-2') ; 43.74 ;
(C-6') ; 157.69 (c = 0) ppm.

Beispiel 3

2',3,3",6'-Tetra-N-(o-nitrophenylsulfenyl)-sisomicin

3a) Penta-N-(o-nitrophenylsulfenyl)-sisomicin

13,84 g (20 mMol) Sisomicinsulfat in 100 ml 1 n NaOH und 450 ml frisch destilliertes Dioxan werden mit 38 g (0,10 Mol) o-Nitrophenylsulfenylchlorid in 200 ml Dioxan und mit 260 ml 1 n-NaOH versetzt, so daß der pH zwischen 12 und 14 liegt. Der Niederschlag wird abfiltriert, in $CH_2Cl_2/H_2O$ gelöst, und die $CH_2Cl_2$-Phase mit $Na_2SO_4$ getrocknet.

Das Filtrat wird mit $CH_2Cl_2$ versetzt, die wäßrige Phase verworfen und die organische Phase über $Na_2SO_4$ getrocknet. Die vereinigten organischen Phasen werden zur Trockne eingedampft und über

250 g Kieselgel (Säulendurchmesser 8 cm) zuerst mit $CH_2Cl_2$, dann mit $CH_2Cl_2$/MeOH = 97,5/2,5 filtriert. Das Eluat liefert nach dem Abdampfen des Lösungsmittels 22 g (91 %) Penta-N-(o-nitrophenylsulfenyl)-sisomicin als orangefarbenem Schaum.

13-C-NMR ($CDCl_3$) ;
δ = 124-148 (arom. H) ;
102.30 (– 1″) ; 99.00 (C-1′) ;
97.91 (C-4′) ; 89.05 (C-6′) ;
82.33 (C-4) ; 57.31 (C-1) ;
56.73 (C-3) ppm.

3b) 3″-N-(o-Nitrophenylsulfenyl)-sisomicin

16,0 g (13,2 mMol) Penta-N-NPS-sisomicin (NPS steht für O-Nitrophenylsulfenyl in 80 ml absol. Pyridin werden mit 160 ml Thiophenol versetzt, nach 1 Stunde auf 500 ml Diäthyläther gegossen, der Niederschlag in Dichlormethan/Methanol = 8/2 aufgenommen und über Kieselgel filtriert (Säule 5, 5 × 12 cm, Laufmittel Dichlormethan/Methanol = 8/2, steigender Zusatz des Laufmittelgemisches Methanol/Dichlormethan/20 proz. Ammoniak = 4/2/1) ; die rote Zone liefert nach dem Abdampfen des Lösungsmittels 6,6 g (83 %) 3″-N-o-Nitrophenylsulfenylsisomicin als tiefroten Schaum.

13-C-NMR($CD_3OD$) ;
33.59 ($CH_3N$) ; 52.23 (C-1) ;
51.16 (C-3) ; 53 (C-2′) ;
43.84 (C-6′) ppm.

3c) 2′,3,3″,6′-Tetra-N-(o-nitrophenylsulfenyl)-sisomicin und 1,2′,3″,6′-Tetra-N-(o-nitrophenylsulfenyl)-sisomicin

3,0 g (5,0 mMol) 3″-N-NPS-sisomicin in 5 ml Methanol und 45 ml Dichlormethan werden mit 4,4 g (15,0 mMol) o-Nitrophenylsulfensäure-p-nitrophenylester in 85 ml Eichlormethan versetzt, das Reaktionsgemisch sofort zur Trockne eingedampft, in Dichlormethan aufgenommen und an Kieselgel (Säule 5,5 × 30 cm) mit 200 ml Dichlormethan, dann mit Dichlormethan/Methanol chromatographiert. Es werden 500 Fraktionen aufgefangen, wobei aus den vereinigten Fraktionen 150 bis 250 das 1,2′,3″,6′-Tetra-NPS-sisomicin und aus den Fraktionen 270 bis 500 das 2′,3,3″,6′-Tetra-NPS-Derivat jeweils als orangefarbener Schaum erhalten wird.

1,2′,3″,6′-Tetra-NPS-sisomicin :
$R_F$ ($CH_2Cl_2/CH_3OH$ = 9/1) : 0.62 ;
IR(KBr) : 1 501, 1 360, 1,300 (stark) ; 1 587, 1 562, 755 (mittel) ; 1 442, 780, 890 (schwach).

2′,3,3″,6′-Tetra-NPS-sisomicin :
$R_F$($CH_2CH/CH_3OH$ = 9/1) : 0,42 ;
IR (KBr) : 1 500, 1 358, 1 296 (stark) ; 1 586, 1 560, 753 (Mittel) ; 1 442, 890, 779 (schwach).

Beispiel 4

3″-N-(o-nitrophenylsulfenyl)-2′,3,6′-tris-N-trichloracetyl-sisomicin

Beispiel 4a

Penta-N-(o-nitrophenylsulfenyl)-2″-o-[dimethyl-(1,2-dimethyl-propyl-silyl]-sisomicin

60,6 g rohes Penta-N-(o-nitrophenylsulfenyl)-sisomicin und 8,75 g Imidazol werden in 250 ml absolutem Dichlormethan gelöst. Bei 0 °C werden unter Feuchtigkeitsausschluß 22,5 ml Dimethyl-(1,2-dimethyl-propyl)-silylchlorid zugetropft. Der Ansatz wird im Vakuum auf ca. 170 ml eingedampft und bei Raumtemperatur 48 Stunden stehen gelassen. Nach Zugabe von 130 ml absolutem Dichlormethan wird der Niederschlag abgesaugt, das Filtrat mit 350 ml Petroläther kräftig durchgeschüttelt und die Petrolätherphase abdekantiert und verworfen. Das ausgefallene Öl wird in 100 ml Dichlormethan gelöst, mit 250 ml Petroläther erneut ausgefällt und schließlich im Hochvakuum getrocknet. Ausbeute 60 g (89 %) Rohprodukt, das ohne weitere Reinigung für die weiteren Umsetzungen eingesetzt wird. Ein reines Präparat wird durch Chromatographie an Kieselgel mit $CH_2Cl_2/CH_3OH$ = 99/1 erhalten.

$R_F$ ($CH_2Cl_2/CH_3OH$ = 99,5/0,5) : 0,62 ;
13-C-NMR ($CDCl_3$) ;
δ = 124-138 (arom. C) ;
147,54 (C-5′) ; 102,26 (C-1″) ;
97,81 (C-4′) ; 99,09 (C-1′) ;

$-2{,}9$ bis $-3{,}0$ (Si—$CH_3$) ;
22,77 (Si—CH—) ; 30,60 (Si—CH—CH) ppm.

Als Nebenprodukt wird das Penta-N-(o-nitrophenylsulfenyl)-2'',5- bis -o-[dimethyl-(1,2-dimethyl-propyl)-silyl]-sisomicin isoliert.

$R_F$ ($CH_2Cl_2$/$CH_3OH$ = 99,5/0,5) : 0,79 ;
13-C-NMR ($CDCl_3$) ;
= 124-146 (arom. C) ;
148,00 (C-5') ; 96,13 (C-4') ppm.

Beispiel 4b

1,3''-Bis-N-(o-nitrophenylsulfenyl)-2''-o-[dimethyl-(1,2-dimethyl-propyl)-silyl]-sisomicin

56 g rohes Penta-N-(o-nitrophenylsulfenyl)-2''-o-[dimethyl-(1,2-dimethyl-propyl)-silyl]-sisomicin in 36 ml Dichlormethan/70 ml Methanol werden mit 16 g 2-Mercaptobenzthiazol versetzt, geschüttelt, bis klare Lösung erfolgt und bei 5 °C 2 Stunden stehen gelassen. Der sich dabei abscheidende Niederschlag wird abfiltriert und die Lösung ohne Isolierung des gewünschten Produktes für die weiteren Umsetzungen verwendet. Die Ausbeute beträgt etwa 80 % der Theorie. Zur Darstellung eines reinen Präparats wird das Filtrat rasch im Vakuum eingedampft und der Rückstand mit a) Dichlormethan, b) Dichlormethan $CH_3OH$ (8 : 2) und c) mit $CH_2Cl_2$/$CH_3OH$/ 20 % wäßrigem Ammoniak (7 : 2,7 : 0,3), an Kieselgel chromatographiert. Die Ausbeute an reinem Produkt beträgt 25,3 g (69 %)

$R_F$ ($CH_2Cl_2$/$CH_3OH$/20 % wäßriges $NH_3$ = 7 : 2,7 : 0,3) = 0,66 ;
13-C-NMR ($CD_3OD$) ;
$\delta$ = 1,5 (Si—$CH_3$) ; 122-146 (arom. C) ;
147,14 (C-5') ; 103,31 (C-1'') ; 100,16 (C-1') ;
99,30 (C-4') ppm.

Beispiel 4c

1,3''-Bis-N-(o-nitrophenylsulfenyl)-2',3,6'-tris-N-trichloracetyl-2''-o-[dimethyl-(1,2-dimethyl-propyl)-silyl]-sisomicin

8,8 g 1,3''-Bis-N-(o-nitrophenylsulfenyl)-2''-o-[dimethyl-(1,2-dimethyl-propyl)-silyl]-sisomicin in 20 ml Dichlormethan/20 ml Pyridin werden bei − 15 °C tropfenweise mit 7,5 ml Trichloressigsäureanhydrid versetzt und noch 10 Minuten bei Raumtemperatur weitergerührt. Nach Zugabe von 20 ml Dichlormethan wird der Ansatz zweimal mit je 20 ml $H_2O$ ausgeschüttelt, die organische Phase eingedampft und der Rückstand als Rohprodukt weiterverarbeitet. $R_F$ ($CH_2Cl_2$/$CH_3OH$ = 97,5/2,5) = 0,72

Beispiel 4d

1,3''-Bis-N-(o-nitrophenylsulfenyl)-2',3,6'-tris-N-trichloracetyl-sisomicin

Das rohe Öl von Beispiel 4c wird in 20 ml Dimethylsulfoxid gelöst, mit 2 ml einer 50 %igen KF-Lösung versetzt und 3 Stunden kräftig gerührt. Das Produkt wird mit Wasser ausgefällt, mit Wasser gewaschen und getrocknet. Das Rohprodukt wird ohne weitere Reinigung weiterverarbeitet.

$R_F$ ($CH_2Cl_2$/$CH_3OH$ = 97,5/2,5) = 0,42 ;
13-C-NMR ($CDCl_3$) ;
$\delta$ = 103,60 (C-1'') ; 66,48 (C-3'') ; 55,15 (C-1) ;
50,60 (C-3) ; 79,86 (C-4) ; 76,18 (C-5) ;
89,16 (C-6) ; 97,74 (C-1') ; 96,84 (C-4') ;
149,80 (C-5) ; 92,78 ($CCl_3$) ; 162,29 und
162,11 (CO) ppm.

3''-N-(o-nitrophenylsulfenyl)-2',3,6'-tris-N-trichloracetyl-sisomicin

Das Produkt von Beispiel 4d wird in 13 ml Dichlormethan gelöst, mit 26 ml Methanol und 5 g 2-Mercaptobenzthiazol bis zur klaren Lösung geschüttelt und 3 Tage bei 5 °C stehen gelassen. Der Niederschlag wird abfiltriert, das Filtrat eingedampft und an Kieselgel chromatographiert.
(Laufmittel a : $CH_2Cl_2$/$CH_3OH$ = 95/5 ; b : ($CH_2Cl_2$/$CH_3OH$/20 % wäßriges $NH_3$ = 93/6,5/0,5).

$R_F$ : ($CH_2Cl_2$/$CH_3OH$/20 % wäßriges $NH_3$ = 93/6,5/0,5) : 0,43 ;
13-C-NMR ($CDCl_3$) ;

δ = 103,43 (C-1″) ; 67,46 (C-3″) ; 50,85 (C-1) ;
50,28 (C-3) ; 79,44 (C-4) ; 76,51 (C-5) ;
89,29 (C-6) ; 97,61 (C-1′) ; 96,62 (C-4′) ;
149,50 (C-5′) 92,46 und 92,38 (C-4′) ;
162,01 und 161,76 (CO) ppm.

Beispiel 5

3″-N-(o-nitrophenylsulfenyl)-2′,3,6′-tris-N-acetyl-sisomicin

Beispiel 5a

1,3″-Bis-N-(o-nitrophenylsulfenyl)-2′,3,6′-triacetyl-2″-o-[dimethyl-(1,2-dimethyl-propyl)-silyl]-sisomicin

Die rohe Lösung von 1,3″-Bis-N-(o-nitrophenylsulfenyl)-2″-o-[dimethyl-(1,2-dimethyl-propyl)-silyl]-sisomicin wird innerhalb von 2 Minuten unter Eiskühlung mit 15 ml Acetanhydrid und 38 ml 6 n NaOH so versetzt, daß das Reaktionsgemisch stets alkalisch bleibt. Der Ansatz wird im Vakuum eingedampft, bis das rote Öl sich klar absetzt. Die wäßrige Phase wird abdekantiert und das Öl mit 120 ml $H_2O$ im Vakuum bei 30-40 °C so durchgerührt, daß ein Teil (ca. 20-30 ml) des Wassers abdestilliert. Die Wasserphase wird abdekantiert und das Öl ohne Reinigung weiterverarbeitet. Chromatographie an Kieselgel mit $CH_2Cl_2/CH_3OH$ = 95/5 liefert ein reines Präparat.

$R_F$ ($CH_2/Cl_2/CH_3OH$ = 95/5) : 0,18 ;
($CH_2Cl_2/CH_3OH$ = 90/10) : 0,7 ;
H-NMR (220 MHz) ;
δ = 2,02, 1,92, 1,89 ($CH_3$—CO) ;
3,03 (N—$CH_3$) ppm.

Beispiel 5b

1,3″-Bis-N-(o-nitrophenylsulfenyl)-2′,3,6′-N-triacetyl-sisomicin

a) Ausgehend von 1,3″-Bis-N-(o-nitrophenylsulfenyl)-2′,3,6′-triacetyl-2″-o-[dimethyl-(1,2-dimethyl-propyl)-silyl]-sisomicin

Das nach Beispiel 5a erhaltene ungereinigte Öl wird in 100 ml Dimethylsulfoxid (DMSO) gelöst, mit 8,75 ml einer 50 %igen wäßrigen KF-Lösung versetzt und kräftig gerührt. Nach 2 Stunden wird der Ansatz auf 300 g Eis gegossen, der Niederschlag absitzen lassen und die wäßrige DMSO-Phase vorsichtig dekantiert. Der Rückstand wird mit 100 ml Wasser verrührt, die wäßrige Phase dekantiert und der Rückstand im Hochvakuum getrocknet. Man erhält 28 g (76 % bezogen auf Penta-N-(O-nitrophenylsulfenyl)-2″-o-[dimethyl-(1,2-dimethylpropyl)-silyl]-sisomicin nach Beispiel 4a) rohes Produkt, welches durch Chromatographie mit $CH_2Cl_2/CH_3OH$ = 95/5 an Kieselgel weiter gereinigt werden kann.

3″-N-(o-nitrophenylsulfenyl)-2′,3,6′-tris-N-acetyl-sisomicin

28 g Rohprodukt von Beispiel 5b werden in 56 ml Dichlormethan gelöst, mit 18 g 2-Mercaptobenzthiazol sowie 93 ml Methanol versetzt und kräftig geschüttelt, bis eine klare Lösung entstanden ist. Der Ansatz wird 20 Stunden bei 5 °C gehalten und tropfenweise mit 16 ml 12 %iger $H_2O_2$-Lösung versetzt. Der Niederschlag wird abgesaugt und das Filtrat im Vakuum eingedampft. Man erhält einen roten Schaum, der für die meisten Folgeumsetzungen ohne weitere Reinigung eingesetzt werden kann. Die Ausbeute beträgt 26,8 g (88,6 %, bezogen auf rohes Penta-N-(o-nitrophenylsulfenyl)-2″-o-[dimethyl-(1,2-dimethylpropyl)-silyl]-sisomicin nach Beispiel 4a). Der Schaum besteht zu 57 % aus dem gewünschten Produkt, d. h. die Gesamtausbeute, bezogen auf Beispiel 4a beträgt 51 % der Theorie. Zur weiteren Reinigung werden 3 g des Rohproduktes an Kieselgel [Säule 3,8 × 30 cm, Laufmittel $CH_2Cl_2/CH_3OH$ = 9/1 mit steigendem Zusatz (zuletzt 5 %) einer Mischung aus $CH_2Cl_2/CH_3OH$/20 % wäßriges $NH_3$ = 2/4/1] chromatographiert. Die Ausbeute ist 1,4 g.

$R_F$ ($CH_2Cl_2/CH_3OH$/20 % wäßriges $NH_3$ = 7,5 : 2,4 : 0,15) : 0,79 ;
13-C-NMR (d6-DMSO) :
71,70 (C-2″) ; 50,89 (C-1) ; 47,73 (C-3) ; 80,95 (C-4) ;
74,79 (C-5) ; 86,53 (C-6) ; 45,52 (C-2′) : 96,55 (C-1′) ;
146,78 (C-5′) ; 169,23, 168,94, 169,64 (3 × CO) ppm.

In den folgenden Ausführungsbeispielen wurden zur Ermittlung des Rf-Wertes die folgenden Laufmittelsysteme benutzt :

Laufmittelsystem G = Methylenchlorid : Methanol : 15 %iger wäßriger Ammoniak (1 : 1 : 1), davon die

Unterphase mit Zusatz von 1 % Methanol

Laufmittelsystem E = Methylenchlorid : Methanol : 20 %iger wäßriger Ammoniak (2 : 4 : 1)

Laufmittelsystem P = Chloroform : Methanol : Aceton : 20 %iger wäßriger Ammoniak (2 : 2 : 1 : 1)

Laufmittelsystem B = Methylenchlorid : Methanol : 20 %iger wäßriger Ammoniak (5 : 5 : 1)

Die Dünnschichtchromatographie erfolgte auf Kieselgelfertigplatten der Firma E. Merck, Darmstadt. Für die Säulentrennungen wurde Kieselgel 60 der Firma E. Merck verwendet.

Beispiel 6

1-N-[(S,R,S,R)-2-ethyloxycarbonylamino-3,4,5,6-tetrahydroxyhexyl]-3″-N-(o-nitrophenylsulfenyl)-2′-3,6′-tri-N-trichloracetyl-sisomicin

Man löst 600 mg 3″-N-(o-nitrophenylsulfenyl)-2′,3,6′-tri-N-trichloracetyl-sisomicin und 200 mg 2-Ethyloxycarbonylamino-2-desoxy-D-glucose in 6 ml Methanol und 1 ml Wasser und rührt, nachdem man die Mischung durch Zugabe von 50 %iger Essigsäure auf pH 5,5 gestellt hat, bei Raumtemperatur für 30 Minuten. Dann fügt man 200 mg Natriumcyanoborhydrid zu und erhitzt für fünf Stunden auf 60 °C.

Zur Aufarbeitung wird das Lösungsmittel im Vakuum abgedampft. Man nimmt in 2 ml Methanol und 10 ml Methylenchlorid auf und wäscht mit 10 ml Wasser. Die organische Phase wird mit Natriumsulfat getrocknet. Sie enthält die gewünschte Verbindung als Hauptprodukt.

Rf-Wert = 0,57 (Laufmittelsystem G)

Beispiel 7

1-N-[(S,R,S,R)-2-ethyloxycarbonylamino-3,4,5,6-tetrahydroxyhexyl]-3″-N-(o-nitrophenylsulfenyl)-sisomicin

Zur Abspaltung der Trichloracetylgruppen löst man den Feststoff von Beispiel 6 in 9 ml Methanol und 3 ml Wasser und erhitzt nach Zugabe von 540 mg Bariumhydroxidoctahydrat für 7 Stunden auf 50 °C. Man entfernt die Bariumsalze durch Fällen mit $CO_2$ und Abfiltrieren, dampft das Filtrat im Vakuum ein und isoliert die gewünschte Verbindung durch Säulenchromatographie an Kieselgel mit dem Lösungsmittelgemisch E als Elutionsmittel. Durch Vereinigen der entsprechenden Fraktionen erhält man die reine gewünschte Verbindung als gelben Feststoff.

Rf-Wert = 0,29 (Laufmittelsystem E)

Beispiel 8

1-N-[(S,R,S,R)-2-ethyloxycarbonylamino-3,4,5,6-tetrahydroxyhexyl]-sisomicin

Man löst das Produkt von Beispiel 7 in 5 ml Methylchlorid und 2,5 ml Methanol versetzt mit 1 ml einer Lösung von 850 mg 2-Mercaptobenzthiazol in 3 ml Methanol und 5 ml Methylenchlorid und säuert mit Salzsäure bis pH 1 an. Dann fügt man 3 ml Wasser zu und schüttelt gut durch. Die Wasserphase, die das gewünschte Produkt enthält, wird abgetrennt und noch zweimal mit je 2 ml Methylenchlorid gewaschen. Dann stellt man durch Rühren mit basischem Ionenaustauscherharz (Lewatit MP $500^R$, BAYER, $OH^{\ominus}$-Form) alkalisch, rührt mit Aktivkohle aus, filtriert und dampft das Filtrat im Vakuum zum amorphen Feststoffen.

$R_f$-Wert = 0,34 (Laufmittelsystem P)

Beispiel 9

1-N-[(R,R,S,R)-2-acetylamino-3,4,5,6-tetrahydroxy-hexyl]-3″-N-(o-nitrophenylsulfenyl)-2′,3,6′-tri-N-trichloracetyl-sisomicin

Man löst 600 mg 3″-N-(o-nitrophenylsulfenyl)-2′,3,6′-tris-N-trichloracetyl-sisomicin und 200 mg 2-Acetylamino-2-desoxy-D-mannose in 6 ml Methanol und 1 ml Wasser und rührt, nachdem man die Mischung durch Zugabe von 50 %iger Essigsäure auf pH 5,5 gestellt hat, bei Raumtemperatur für 30 Minuten. Dann fügt man 200 mg Natriumcyanoborhydrid zu und erhitzt für fünf Stunden auf 60 °C.

Zur Aufarbeitung wird das Lösungsmittel im Vakuum abgedampft. Man nimmt in 2 ml Methanol und 10 ml Methylenchlorid auf und wäscht mit 10 ml Wasser. Die organische Phase wird mit Natriumsulfat getrocknet. Sie enthält die gewünschte Verbindung als Hauptprodukt.

$R_f$-Wert = 0,48 (Laufmittelsystem G)

Beispiel 10

1-N-[(R,R,S,R)-2-acetylamino-3,4,5,6-tetrahydroxy-hexyl]-3″-N-(o-nitrophenylsulfenyl)-sisomicin

Zur Abspaltung der Trichloracetylgruppen löst man den Feststoff von Beispiel 9 in 9 ml Methanol und 3 ml Wasser und erhitzt nach Zugabe von 540 mg Bariumhydroxidoctahydrat für 7 Stunden auf 50 °C. Man entfernt die Bariumsalze durch Fällen mit $CO_2$ und Abfiltrieren, dampft das Filtrat im Vakuum ein und isoliert die gewünschte Verbindung durch Säulenchromatographie an Kieselgel, mit dem Lösungsmittelgemisch E als Elutionsmittel. Durch Vereinigen oder entsprechenden Fraktionen erhält man die reine gewünschte Verbindung als gelben Feststoff.

$R_f$-Wert = 0,32 (Laufmittelsystem E)

Beispiel 11

1-N[(R,R,S,R)-2-acetylamino-3,4,5,6-tetrahydroxyhexyl]-sisomicin

Man löst das Produkt von Beispiel 10 in 5 ml Methylenchlorid und 2,5 ml Methanol versetzt mit 1 ml einer Lösung von 850 mg 2-Mercaptobenzthiazol in 3 ml Methanol und 5 ml Methylenchlorid und säuert mit Salzsäure bis pH 1 an. Dann fügt man 3 ml Wasser zu und schüttelt gut durch. Die Wasserphase, die das gewünschte Produkt enthält wird abgetrennt und noch zweimal mit je 2 ml Methylenchlorid gewaschen. Dann stellt man durch Rühren mit basischen Ionenaustauscherharz (Lewatit MP 500$^R$, BAYER, $OH^{\ominus}$-Form) auf pH 11,5, rührt mit Aktivkohle aus, filtriert und dampft das Filtrat im Vakuum zum amorphen Feststoffen ein.

$R_f$-Wert = 0,32 (Laufmittelsystem P)

Beispiel 12

1-N-[(S,R,R,R)-5-amino-2,3,4,6-tetrahydroxyhexyl]-sisomicin

200 mg 3″-N-(o-nitrophenylsulfenyl)-2′,3,6′-tri-N-acetyl-sisomicin und 200 mg 5-Acetylamino-5-desoxy-D-glucose werden in 7 ml Methanol und 1,4 ml Wasser für 30 Minuten auf 70 °C erhitzt. Dann fügt man 70 mg Natriumcyanoborhydrid zu und erhitzt weitere 60 Minuten. Man läßt dann auf Raumtemperatur abkühlen, fügt 5 ml Wasser zu und entionisiert mit basischen Ionenaustauscherharz (Lewatit MP 500$^R$, $OH^{\ominus}$-Form, BAYER AG, Leverkusen). Nach Filtrieren wird das Filtrat im Vakuum vom Lösungsmittel befreit.

Zur Abspaltung der Schutzgruppen löst man den oben erhaltenen Rückstand in 6 ml Wasser und erhitzt nach Zusatz von 3,6 g Bariumhydroxidoctahydrat für 5 Stunden am Rückfluß. Die Bariumionen werden anschließend als Bariumsulfat ausgefällt. Man filtriert, entionisiert das Filtrat mit basischen Austauscherharz ($OH^{\ominus}$-Form, s.o.), filtriert erneut und dampft das Filtrat im Vakuum zur Trockne ein.

Man erhält so die gewünschte Verbindung als amorphen Feststoff.

$R_f$-Wert = 0,33 (Laufmittelsystem B)

Beispiel 13

1-N-[(S,R,R,R)-5-methylamino-2,3,4,6-tetrahydroxyhexyl]-sisomicin

300 mg 3″-N-(o-nitrophenyl)-2′, 3′, 6′-tri-N-acetyl-sisomicin und 5-Methylamino-5-desoxy-N-acetyl-D-glucose werden in 10 ml Methanol + 2,5 ml Wasser für 30 Minuten auf 60 °C erhitzt. Dann fügt man 100 mg Natriumcyanoborhydrid zu und erhitzt weitere 90 Minuten. Nach läßt auf Raumtemperatur kommen, fügt 8 ml Wasser zu und entionisiert mit basischem Ionenaustauscherharz. Man filtriert und dampft im Vakuum das Lösungsmittel ab. Der Rückstand wird in 5 ml Methanol und 5 ml Methylenchlorid gelöst, und man versetzt mit 1 ml einer Lösung von 850 mg 2-Mercaptobenz-thiazol in 3 ml Methanol und 5 ml Methylenchlorid und säuert mit Salzsäure auf pH 1 an. Dann fügt man 10 ml Wasser zu und schüttelt gut durch. Die Wasserphase — in der sich das gewünschte N-desulfenylierte Produkt befindet — wird mit basischem Ionenaustauscherharz alkalisch gemacht. Man filtriert vom Harz ab und erhitzt das so erhaltene Filtrat zur Abspaltung der N-acetylgruppen nach Zusatz von 4 g Bariumhydroxidoctahydrat für 5 Stunden am Rückfluß. Die Bariumionen werden als Bariumsulfat ausgefällt, was man abfiltriert. Das Filtrat wird mit basischem Ionenaustauscher entionisiert. Nach Filtrieren verdampft man das Lösungsmittel im Vakuum und erhält die Titelverbindung als amorphen Feststoff.

$R_f$-Wert = 0,35 (Laufmittelsystem B)

**Ansprüche**

1. Pseudotrisaccharide der Formel

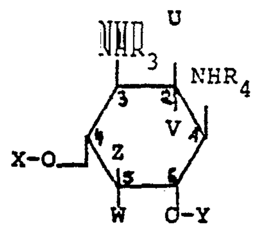

in der

X einen Rest

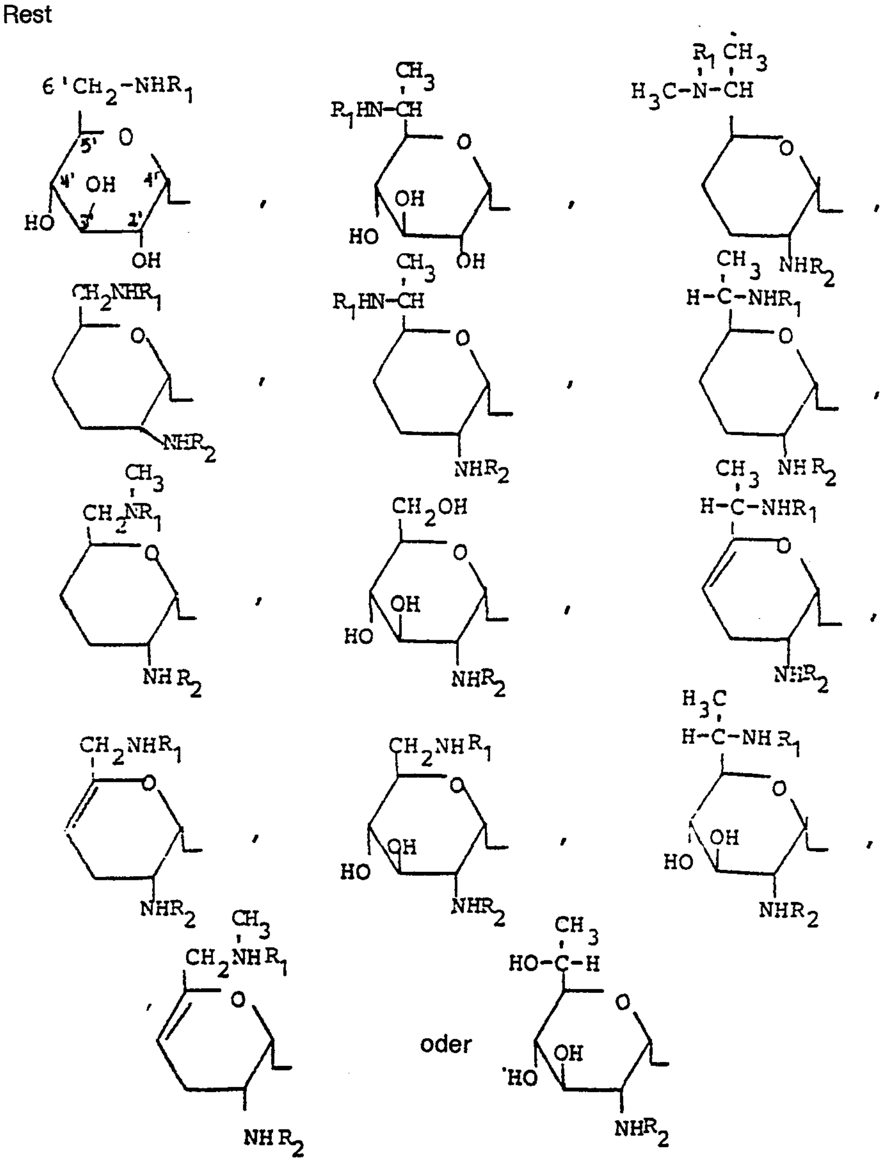

Y einen Rest

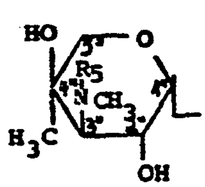 , 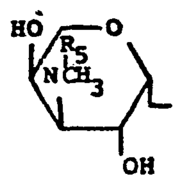 oder 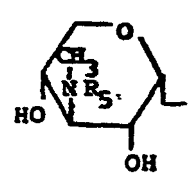

U und V beide Wasserstoff oder einer Wasserstoff und einer Hydroxy,

W und Z beide Wasserstoff oder einer Wasserstoff und einer Hydroxy oder Z $NHR_6$ und W Wasserstoff bedeuten,

und worin

$R_1$, $R_2$, $R_3$, $R_4$, $R_5$ und $R_6$ unabhängig voneinander Wasserstoff oder einen durch reduktive Alkylierung in Gegenwart einer Amino-desoxy-aldose eingeführten Aminopolyhydroxyalkylrest bedeuten, der nicht mehr als 2 Aminogruppen enthält und welcher gegebenenfalls am N-Atom acyliert oder

sulphenyliert ist sowie gegebenenfalls an einer oder mehreren OH-Gruppen Alkyl-, Acyl- oder Alkylidenreste trägt, mit der Bedingung, daß wenigstens einer der Reste $R_1$ bis $R_5$ nicht Wasserstoff ist, sowie deren pharmazeutisch verwendbare Salze.

2. Pseudotrisaccharide gemäß Anspruch 1, die von den Antibiotika Gentamicin A, Gentamicin B, Gentamicin $B_1$, Gentamicin $C_1$, Gentamicin $C_{1a}$, Gentamicin $C_2$, Gentamicin $C_{2a}$, Gentamicin $C_{2b}$, Gentamicin $X_2$, Sisomicin, JI-20A, JI-20B, Verdamicin G52, Mutamicin 1, Mutamicin 2, Mutamicin 4, Mutamicin 5 und Mutamicin 6 abgeleitet sind.

3. Sisomicinderivate der Formel

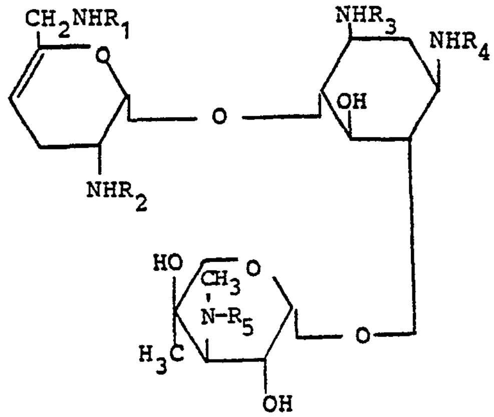

worin $R_1$, $R_2$, $R_3$, $R_4$ und $R_5$ die in Anspruch 1 angegebene Bedeutung besitzen.

4. Pseudotrisaccharide gemäß Anspruch 1 bis 3, in denen $R_4$ und einer der Reste $R_1$ und $R_2$ nicht Wasserstoff sind, und die Reste $R_3$, $R_5$, $R_6$ und der andere der Reste $R_1$ und $R_2$ Wasserstoff bedeuten.

5. Verfahren zur Herstellung von Pseudotrisacchariden gemäß Anspruch 1, dadurch gekennzeichnet, daß man Verbindungen der Formel

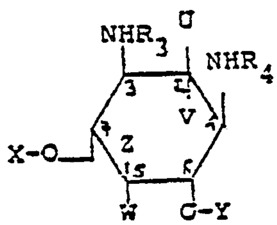

worin

$R_1$, $R_2$, $R_3$, $R_4$, $R_5$ und $R_6$ Wasserstoff, —SR' oder —CO—A' bezeichnen mit der Maßgabe, daß wenigstens einer der Reste $R_1$ bis $R_6$ Wasserstoff bedeutet und wenigstens einer der Reste $R_1$ bis $R_6$ —SR' oder —CO—A' bedeutet, wobei R' gegebenenfalls substituiertes Phenyl, Di- oder Triphenylmethyl und A'

$C\,Hal_3$, $-(CH_2)_{n_6}-D$ oder 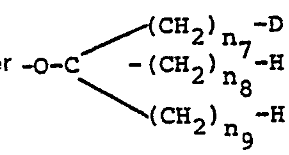

bedeuten, worin Hal für Fluor, Chlor oder Brom

D für Wasserstoff oder gegebenenfalls substituiertes Phenyl und

$n_6$, $n_7$, $n_8$ und $n_9$ unabhängig voneinander der für eine Zahl von 0 bis 5 stehen, und worin

U, V, W, Z, X und Y die in Anspruch 1 angegebene Bedeutung haben, mit einem Aminozucker, welcher gegebenenfalls am N-Atom acyliert oder sulphenyliert ist, sowie gegebenenfalls an einer oder mehreren OH-Gruppen (außer an der 1-OH-Gruppe) Alkyl-, Acyl- oder Alkylidenreste trägt, in Gegenwart eines Wasserstoffdonor-Reduktionsmittels umsetzt und anschließend die Schutzgruppen —SR' und —COA' und gegebenenfalls Acylgruppen abspaltet.

6. Verfahren nach Anspruch 5, dadurch gekennzeichnet, daß der Aminozucker, bzw. das Aminozuckerderivat in der Aldehydform eingesetzt wird.

7. Verfahren nach Anspruch 5, dadurch gekennzeichnet, daß der Aminozucker bzw. das Aminozuckerderivat in acetalisierter Form eingesetzt wird.

8. Arzneimittel, gekennzeichnet durch einen Gehalt an einer Verbindung gemäß Anspruch 1.

9. Verbindungen gemäß Anspruch 1 zur Bekämpfung von durch Bakterien hervorgerufenen Erkrankungen.

**Claims**

1. Pseudotrisaccharides of the formula

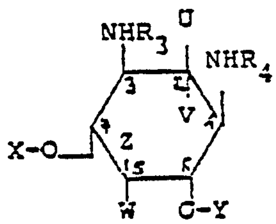

in which

X denotes a radical

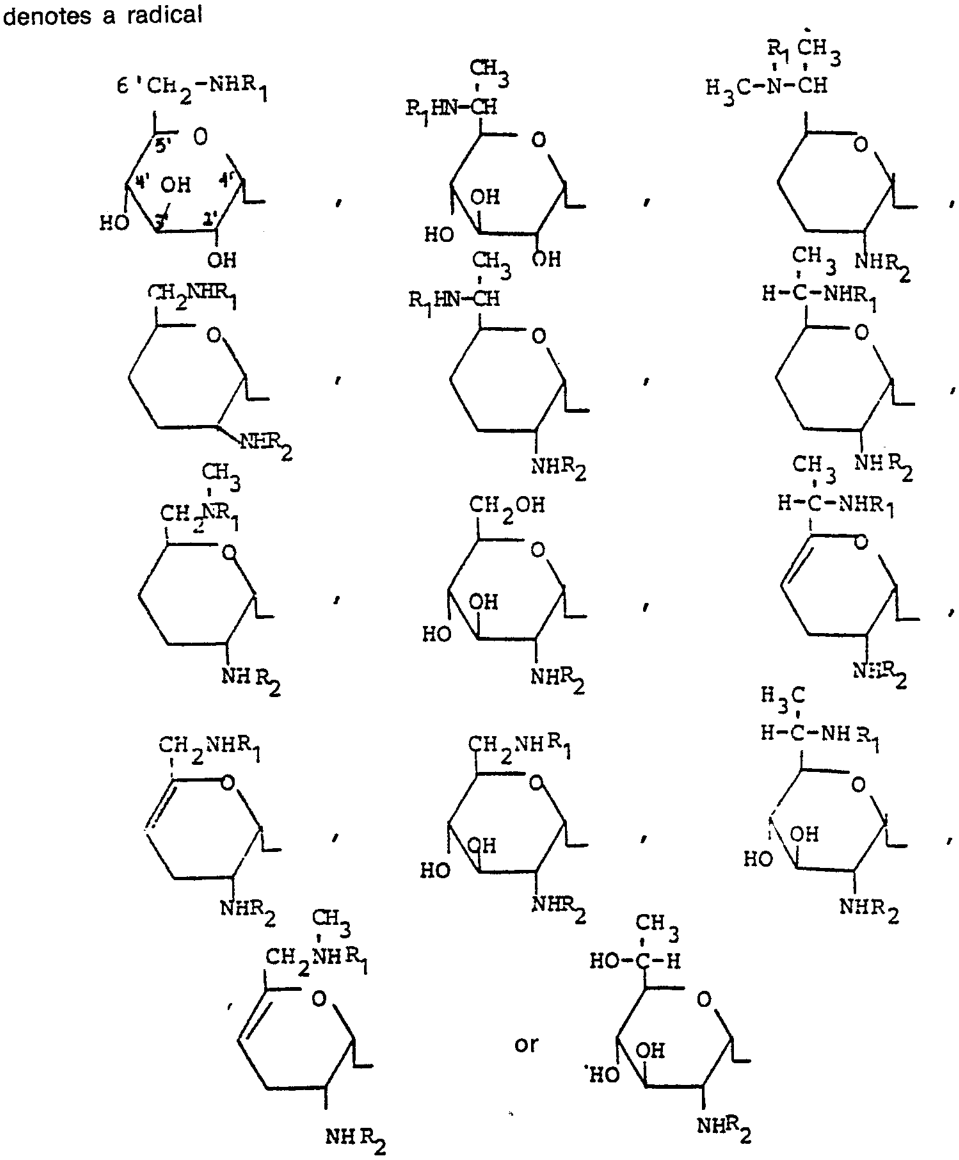

, , , , , , , , , , , , or

Y denotes a radical

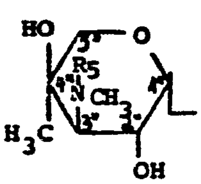

,

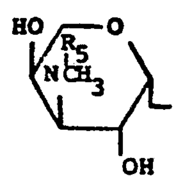

or

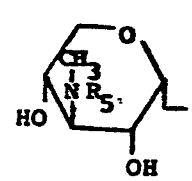

U and V both denote hydrogen, or one denotes hydrogen and one denotes hydroxyl and

W and Z both denote hydrogen, or one denotes hydrogen and one denotes hydroxyl, or Z denotes $NHR_6$ and W denotes hydrogen,
and wherein

$R_1$, $R_2$, $R_3$, $R_4$, $R_5$ and $R_6$ independently of one another denote hydrogen or an aminopolyhydroxyalkyl radical introduced by reductive alkylation, in the presence of an amino-desoxy-aldose which does not contain more than 2 amino groups and which is optionally sulphenylated or acylated on the N atom and optionally carries alkyl, acyl or alkylidene radicals on one or more OH groups, with the condition that at least one of the radicals $R_1$ to $R_5$ is other than hydrogen,
and their pharmaceutically acceptable salts.

2. Pseudotrisaccharides according to Claim 1, which are derived from the antibiotics gentamicin A, gentamicin B, gentamicin $B_1$, gentamicin $C_1$, gentamicin $C_{1a}$, gentamicin $C_2$, gentamicin $C_{2a}$, gentamicin $C_{2b}$, gentamicin $X_2$, sisomicin, JI-20A, JI-20B, verdamicin G52, mutamicin 1, mutamicin 2, mutamicin 4, mutamicin 5 and mutamicin 6.

3. Sisomicin derivatives of the formula

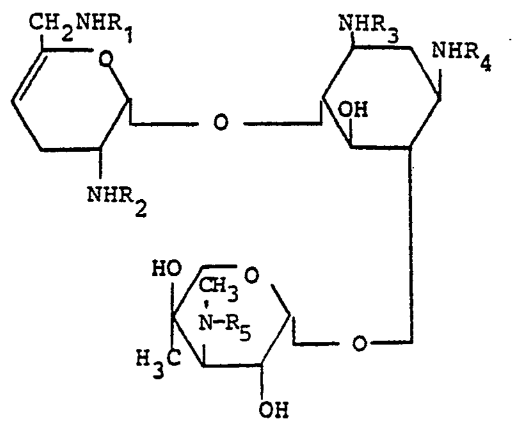

wherein $R_1$, $R_2$, $R_3$, $R_4$ and $R_5$ have the meaning indicated in Claim 1.

4. Pseudotrisaccharides according to Claim 1, in which $R_4$ and one of the radicals $R_1$ and $R_2$ are other than hydrogen and the radicals $R_3$, $R_5$, $R_6$ and the other one of the radicals $R_1$ and $R_2$ denote hydrogen.

5. Process for the preparation of pseudotrisaccharides according to Claim 1, characterised in that compounds of the formula

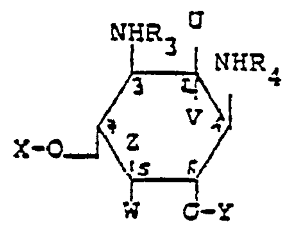

wherein

$R_1$, $R_2$, $R_3$, $R_4$, $R_5$ and $R_6$ designate hydrogen, —SR' or —CO—A', with the proviso that at least one of the radicals $R_1$ to $R_6$ denotes hydrogen and at least one of the radicals $R_1$ to $R_6$ denotes —SR' or —CO—A', wherein R' denotes optionally substituted phenyl or di- or tri-phenylmethyl and A' denotes

$C\ Hal_3$, $-(CH_2)_{n_6}-D$ or 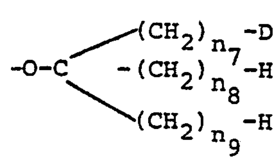

wherein Hal represents fluorine, chlorine or bromine

D represents hydrogen or optionally substituted phenyl, and

$n_6$, $n_7$, $n_8$ and $n_9$ independently of one another represent a number from 0 to 5 and
wherein

U, V, W, Z, X and Y have the meaning indicated in Claim 1, are reacted with an amino-sugar which is optionally sulphenylated or acylated on the N atom and optionally carries alkyl, acyl or alkylidene radicals on one or more OH groups (except on the 1 OH group), in the presence of a hydrogen donor reducing agent, and the protective groups —SR' and —COA' and, if appropriate, acyl groups are then split off.

6. Process according to Claim 5, characterised in that the amino-sugar or the amino-sugar derivative is employed in the form of aldehyde.

7. Process according to Claim 5, characterised in that the amino-sugar or the amino-sugar derivative is employed in the form of an acetal.

8. Medicaments, characterised in that they contain a compound according to Claim 1.

9. Compounds according to Claim 1 for combating illnesses caused by bacteria.

**Revendications**

1. Pseudotrisaccharides de formule

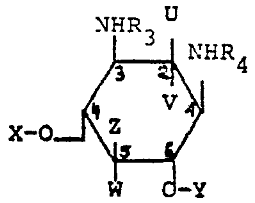

dans laquelle

X est un reste

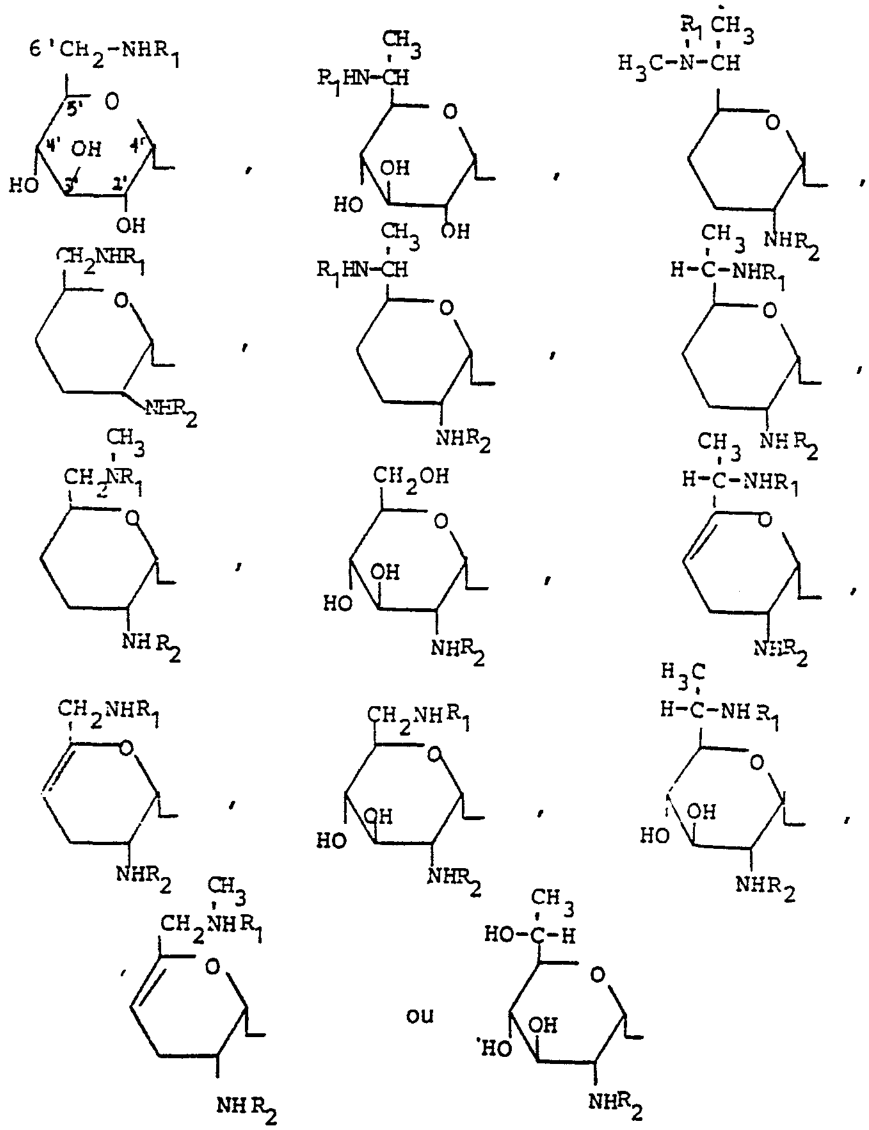

, , , , , , , , , , , , ou

Y est un reste

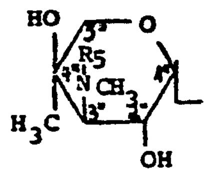

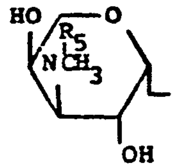

ou

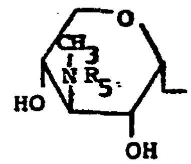

U et V sont tous deux de l'hydrogène ou bien l'un est de l'hydrogène et l'autre est un groupe hydroxy,

W et Z sont tous deux de l'hydrogène ou bien l'un est un atome d'hydrogène et l'autre est un groupe hydroxy ou bien Z est un groupe $NHR_6$ et W est un atome d'hydrogène, et

$R_1$, $R_2$, $R_3$, $R_4$, $R_5$ et $R_6$ représentent, indépendamment les uns des autres, de l'hydrogène ou un reste aminopolyhydroxyalkyle introduit par alkylation par voie de réduction en présence d'un aminodésoxyaldose, qui ne contient pas plus de 2 groupes amino et qui est acylé éventuellement sur l'atome d'azote ou qui est sulfénylé, de même qu'il porte éventuellement sur un ou plusieurs groupes OH des restes alkyle, acyle ou alkylidène, à condition qu'au moins l'un des restes $R_1$ à $R_5$ ne soit pas de l'hydrogène,

de même que leurs sels utilisables en pharmacie.

2. Pseudotrisaccharides suivant la revendication 1, qui sont dérivés des antibiotiques suivants : gentamicine A, gentamicine B, gentamicine $B_1$, gentamicine $C_1$, gentamicine $C_{1a}$, gentamicine $C_2$, gentamicine $C_{2a}$, gentamicine $C_{2b}$, gentamicine $X_2$, sisomicine JI-20A, JI-20B, verdamicine G52, mutamicine 1, mutamicine 2, mutamicine 4, mutamicine 5 et mutamicine 6.

3. Dérivés de sisomicine de formule

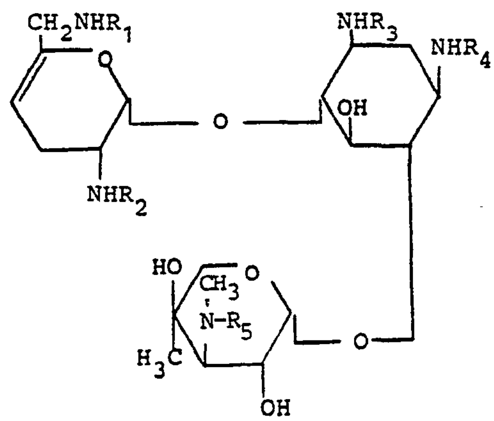

dans laquelle $R_1$, $R_2$, $R_3$, $R_4$ et $R_5$ ont la définition indiquée dans la revendication 1.

4. Pseudotrisaccharides suivant les revendications 1 à 3, dans lesquels $R_4$ et l'un des restes $R_1$ et $R_2$ ne représentent pas de l'hydrogène et les restes $R_3$, $R_5$, $R_6$ et l'autre des restes $R_1$ et $R_2$ désignent de l'hydrogène.

5. Procédé de production de pseudotrisaccharides suivant la revendication 1, caractérisé en ce qu'on fait réagir des composés de formule

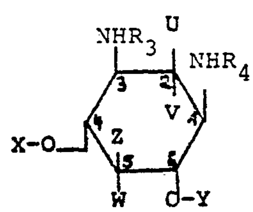

dans laquelle

$R_1$, $R_2$, $R_3$, $R_4$, $R_5$ et $R_6$ représentent de l'hydrogène, un groupe —SR' ou —CO—A', à condition qu'au moins l'un des restes $R_1$ à $R_6$ représente de l'hydrogène et qu'au moins l'un des restes $R_1$ à $R_6$ soit un groupe —SR' ou —CO—A', R' désigne un groupe phényle éventuellement substitué, di- ou triphénylméthyle et A' est un groupe

$C\ Hal_3$, $-(CH_2)_{n_6}-D$ ou 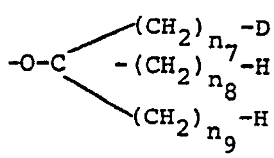

Hal étant le fluor, le chlore ou le brome,

D est l'hydrogène ou un groupe phényle éventuellement substitué et
$n_6$, $n_7$, $n_8$ et $n_9$ représentent, indépendamment les uns des autres, un nombre de 0 à 5, et

U, V, W, Z, X et Y ont la définition donnée dans la revendication 1, avec un amino-sucre qui est éventuellement acylé ou sulfénylé sur l'atome d'azote, de même qu'il porte éventuellement sur un ou plusieurs groupes OH (hormis le groupe OH en position 1) des restes alkyle, acyle ou alkylidène, en présence d'un réducteur donneur d'hydrogène et on élimine ensuite les groupes —SR' et —CO—A' protecteurs et, le cas échéant, les groupes acyle.

6. Procédé suivant la revendication 5, caractérisé en ce que l'amino-sucre ou le dérivé amino-sucre est utilisé sous la forme aldéhydique.

7. Procédé suivant la revendication 5, caractérisé en ce que l'amino-sucre ou le dérivé d'amino-sucre est utilisé sous la forme acétalisée.

8. Médicament, caractérisé en ce qu'il contient un composé suivant la revendication 1.

9. Composés suivant la revendication 1, destinés à combattre des maladies engendrées par des bactéries.